(19) Europäisches Patentamt
European Patent Office
Office européen des brevets

(11) Publication number: 0 232 545 A2

# (12) EUROPEAN PATENT APPLICATION

(21) Application number: 86118044.6

(22) Date of filing: 24.12.86

(51) Int. Cl.[4]: C 08 G 59/68, C 08 G 59/14

(30) Priority: 27.12.85 JP 294605/85

(43) Date of publication of application: 19.08.87 Bulletin 87/34

(84) Designated Contracting States: DE FR GB

(71) Applicant: Nippon Paint Co., Ltd., 2-1-2, Oyodokita Oyodo-ku, Osaka-shi Osaka-fu (JP)

(72) Inventor: Matsumura, Akira, 1-72-15, Kazuhaoka, Hirakata-shi Osaka-fu (JP)
Inventor: Ishikawa, Katsukiyo, 2-40, Sayamakitadai Kumiyamaa-cho, Kuze-gun Kyoto-fu (JP)

(74) Representative: Hansen, Bernd, Dr.rer.nat. et al, Hoffmann, Eitle & Partner Patentanwälte Arabellastrasse 4, D-8000 München 81 (DE)

## (54) Photocurable composition and cured article.

(57) Disclosed is a photocurable composition comprising (a) an epoxy compound modified with a compound having a polymerizable ethylenically unsaturated group, (b) a polymerization initiator capable of initiating cationic polymerization by exposing to light, and (c) a sensitizer and the cured article.

EP 0 232 545 A2

0232545

44 981 u/kü

# PHOTOCURABLE COMPOSITION AND CURED ARTICLE

## Field of the invention

The present invention relates to a photocurable composition curing by exposing to light. Particularly, it relates to a photocurable composition which can be cured to form a thick film having good transparency.

## Background of the invention

Hitherto, ultraviolet-curable compositions have been used for many purposes such as coating, adhesion, ink and so on. The ultraviolet-curable compositions are usually prepared from a (meth)acrylate resin, an unsaturated polyester resin, a polyen-thiol resin, or an epoxy resin. Such ultraviolet-curable compositions have an advantage that cure can be completed in a relarively low temperature for a short period of time, but it is difficult to impart good adhesion properties flexibility, hardness and the like to a cured composition.

In order to improve the above mentioned defects, Japanese Patent Publication (unexamined) 159820/1984 proposes an ultraviolet-curable composition which comprises an epoxy compound, a (meth)acrylate compound, an intiator capable of initiating cationic polymerization by exposing to light and a sensitizer. The composition can be promptly cured by exposing to light and gives a cured composition having good hardness and good addhesion properties flexibility, impact resistance as well as chemical resistance. It, however, has been found that, when the

composition is formed into a thick film, the film loses transparency and develops frosting.

Japanese Patent Publication (unexamined) 159820/1984 explains that the reason why the composition has excellent properties is that the epoxy compounds and the (meth)acrylate compounds are separately polymerized intertwined with each other to form a network. Both components however, inherently have bad compatibility and a complete net work construction is not formed with the result that frosting is developed in thick films.

Summary of the invention

The present invention is to provide a photocurable composition which, when cured to form a thick film, has improved transparency. The photocurable composition comprises:

(a) an epoxy compound modified with a compound having a polymerizable ethylenically unsaturated group (hereinafter referred to as "modified epoxy compound"),

(b) a polymerization initiator capable of initiating cationic polymerization by exposing to light, and

(c) a sensitizer.

According to the present invention, since the modified epoxy compound has an epoxy group and a polymerizable ethylenically unsaturated group, the composition constructs a complete network when cured and therefore develops no frosting.

Detailed description of the present invention

The epoxy compound to be modified with a compound having polymerizable ethylenically unsaturated group is not limited. Representative examples of the epoxy compounds are

bisphenol A type epoxy resins, aliphatic epoxy resins, alicyclic epoxy resins and the like. Most epoxy compounds contain hydroxyl groups through which a modifying reaction is generally conducted. An epoxy compound which does not have hydroxy groups can introduce an active group such as hydroxy group through for modification. For example, two moles of the epoxy compound not having hydroxyl group is reacted with one mole of a dicarboxylic acid, such as azelaic acid under a conventional condition to introduce hydroxyl groups.

An epoxy compound modified with an polymerizable ethylenically unsaturated compound of the present invention can be prepared by reacting the epoxy compound having a hydroxyl group with an isocyanate compound containing a polymerizable ethylenically unsaturated group. Example of the isocyanate compounds are vinyl isocyanate, an isocyanatoalkyl (meth)acrylate (for example, isocyanatoethyl methacrylate and so on), and a (meth)acryloyl isocyanate and the like. The isocyanate compounds containing a polymerizable ethylenically unsaturated group can be prepared by a conventional process. It is preferred that (meth)acryloyl isocyanate is prepared by reacting methacyloyl-amide with oxalyl halide (Japanese Patent Application Ser. No. 225226/1983), or by reacting acrylamide with oxaly halide followed by dehydrohalogenating (Japanese Patent Application Ser. No. 241876/1984). The reaction of the epoxy compound and the isocyanate compound can be carried out by dissolving the epoxy oompound in an inert solvent, refluxing at an elevated temperature, for example 150 °C when xylene is employed, and

adding dropwise the isocyanate compound with stirring under a nitrogen blanket at -20 to +50 °C, preferably 0 to +20 °C. Since the addition of the isocyanate compound is often associated with exotherm, it may be cooled with a water bath. The isocyanate compound can be added neat, but it is preferred to dilute with an inert solvent. The amount of the isocyanate compound is varied depending on a desirable replacing amount of hydroxyl groups. Termination of the reaction can be determined by disappearing NCO absorption in infrared spectrum. The inert solvent mentioned above is one not having an active hydrogen, for example, an aliphatic hydrocarbon such as pentane, heptane and hexane; an aromatic hydrocarbon such as benzene, toluene and xylene; an alicyclic hydrocarbon such as cyclohexane, methylcyclohexane and decaline; a petroleum solvent such as petroleum ether and petroleum benzine; a hydrocarbon halide such as carbon tetrachloride, chloroform and 1,2 -dichloroethane; an ether such as ethyl ether, isopropyl ether, anisole, dioxane and tetrahydrofuran; a ketone such as acetone, methyl ethyl ketone, methyl isobutyl ketone, cyclohexanone, acetophenone and isophorone; an ester such as ethyl acetate, butyl acetate, propylene glycol monomethyl acetate and cellosolve acetate; acetonitrile; dimethylformamide; dimethyl sulfoxide; and the like. Preferred solvents for the present invention are aromatic hydrocarbons. In order to avoid an unnecessary polymerization through double bonds, a polymerization inhibitor may be present in a reaction system. Examples of the polymerization inhibitors are hydroquinone, p-methoxyphenol, 2,6-di-t-methylphenol, 4-t-butylcatechol, bisdihydroxybenzylbenzene, 2,2'-methylenebis(6-t-butyl-3-methylphenol), 4,4'-butylidenebis(6-t-butyl-3-methylphenol), 4,4'-thiobis(6-t-butyl-3-methylphenol), p-nitorosophenol, diisopropylxanthogensulfide, N-nitrosophenylhydroxylamine ammonium salt, 1,1-diphenyl-2-picrylhydrazil, 1,3,5-triphenylpheldazyl, 2,6-di-t-butyl-alpha-(3,5-di-t-butyl-4'-oxo-2,5-cyclohexadiene-1-iriden)-p-trioxy, 2,2,6,6-tetramethyl-4-piperidone-1-oxyl, dithiobenzoylsulfide, p,p'-ditolylsulfide, p,p'-ditolyltetrasulfide, dibenzyltetrasulfide, tetraethylthiuramdisulfide and the like.

Beside a reaction of the epoxy compound and the unsaturated isocyanate, the modified epoxy compound may be prepared by reacting the epoxy compound having hydroxyl groups with a reaction product of a hydroxyalkyl (meth)acrylate with diisocyanate. Preferred hydroxyalkyl (meth)acrylates are those having 1 to 20 carbon atoms in the alkyl group, for example, hydroxymethyl (meth)acrylate, hydroxypropyl (meth)acrylate, hydroxydodecyl (meth)acrylate and the like. Examples of diisocyanates are aliphatic diisocyanates, such as hexamethylene diisocyanate, trimethylhexamethylene diisocyanate, dimer acid diisocyanate and lysine diisocyanate; cycloaliphatic diisocyanates, such as 4,4'-methylenebis(cyclohexyl isocyanate), methylcyclohexane-2,4(2,6)-diisocyanate, 1,3-(isocyanatomethyl)cyclohexane and isophorone diisocyanate; aromatic diisocyanates, such as tolylene diisocyanate, 4,4'-diphenylmethane diisocyanate, xylylene diisocyanate, metaxylylene

diisocyanate; and the like. The modification condition is known to those skilled in the art.

The modified epoxy compound may also be prepared by reacting the epoxy compound having hydroxyl groups with a (meth)acryl halide in the presence of a tertiary amine. The halogen atom may be chlorine, bromine or iodine. The tertiary amines may be either aliphatic amines or aromatic amines. These reaction process are known to the skilled in the art.

The modified epoxy compound of the present invention may be combined with an unmodified epoxy compound. The unmodified epoxy compounds are exemplified in "The Encyclopedia of polymer Science and Technology", Interscience Publisher, New York, 1976, 6, p 209-171.

The polymerization initiator of the present invention can be any one which has capability of intiating cationic polymerization when exposed to light. Preferred are onium compounds which release a material having a capability of initiating cationic polymerization. The onium compounds are represented by following formula:

$$[(R)_a(R^1)_b(R^2)_cX]_d{}^+[MQ_e]^{-(e-f)} \qquad (1)$$

Wherein R represents a monovalent aromatic organic group, $R^1$ represents a monovalent aliphatic organic group selected from an alkyl group, a cycloalkyl group or a substituted alkyl group, $R^2$ represents a polyvalent organic group having heterocyclic or combined cyclic structure, selected from an aliphatic group or an aromatic group, X represents an element selected from diazonium, halogen, nitrogen, arsentic, antimony, bismuth, sulfer, selen or

tellurium, a is an integer of 0 to 3, b is an integer of 0 to 2, c is 0 or 1 and a + b + c is 3 which is equal to a valency of X, d is equal to (e-f), f is an integer of 2 to 7 equal to a valency of M, Q represents a halogen atom and e is an integer up to 5 and more than f. Representative example of R is an aromatic hydrocarbonic group having 4 to 15 carbon atoms, such as phenyl, tolyl, naphthyl, anthryl, arylacyl, benzoyl and phenylacyl; and aromatic heterocyclic group such as pyridyl and furfuryl. R may be substituted with alkoxy groups having 1 to 6 carbon atoms, alkyl groups having 1 to 7 carbon atoms, nitro groups, chlorine, hydroxyl groups. Example of $R^1$ is an alkyl group having 1 to 6 carbon atoms and a substituted alkyl such as $-C_2H_4OCH_3$, $-CH_2COOC_2H_5$, $-CH_2COCH_5$ and the like. Representative example of $R^2$ is

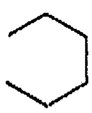 , 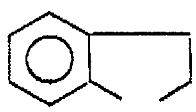 ,

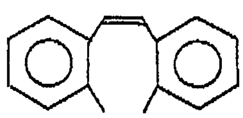 , 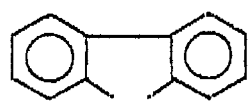

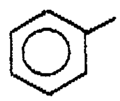 .

Example of $[MQe]^{-(e-f)}$ is $BF_4^-$, $PF_4^-$, $A_3F_4^-$, $SbF_6^-$, $FeCl_4^-$, $SnCl_3^-$, $SbCl_4^-$, $BiCl_5^{2-}$, $AlF_6^{3-}$, $CaCl_4^-$, $InF_4^-$, $TiF_6^{2-}$, $ZrF_6^-$, and the like, wherein M can be transition metals, such as Sb, Fe, Sn, Bi, Al, Ca, In, Ti, Zr, Sc, V, Cr, Mn, Cs; lanthanoids such as Ce, Pr, Nd and so on; actinods such as Th, Pa, U, Np and so on; and semimetals such as B, P, As; and the like.

Beside the onium compound mentioned above, polymerization initiators described in Japanese Patent Publication (unexamined) 219307/1984 may also be employed.

The sensitizers employed in the present invention are, for example, benzoin and an ether thereof, such as methyl ether, ethyl ether, isopropyl ether, butyl ether and octyl ether; a carbonyl compound, such as diacetyl, benzophenone, 2-hydroxy-2-methylpropiophenone, benzyl dimethyl ketal, o-benzoyl methyl benzoate, and 4'-isopropyl-2-hydroxy-2-methyl-propiophenone, an anthraquinone, such as anthroquinone, chloroanthraquinone, ethylanthraquinone and butylanthraquinone; a sulfur compound, such as diphenylsulfide, dithiocarbamate and 2-chlorothioxanthone; alpha-chloromethylnaphtharene; anthracene; and the like. The sensitizer is added to the composition of the present invention to accelerate curing speed and to avoid surface tackness.

The polymerization initiator is present in the composition of the present invention in an amount of 0.01 to 10 parts by weight, preferably 0.1 to 5 parts by weight based on 100 parts by weight of the modified epoxy compound which may contain the unmodified epoxy compound if desired. The sensitizer is present in the composition of the present invention in an amount of 0.01 to 10 parts by weight, preferably 0.1 to 8 parts by weight based on 100 parts by weight of the modified epoxy compound which may include the unmodified epoxy compound if desired. When the epoxy compound which is not modified is combined with the modified epoxy compound, the amount of the

modified epoxy compound is within the range of 95 to 5 parts by weight, preferably 90 to 10 parts by weight based on 100 parts by weight of both epoxy compounds.

The photocurable composition of the present invention may contain additives, such as fillers, dyes, pigments, thickners, thixotropic agents, plastitizer, stabilizer, radical polymerization inhibitors, leveling agents, coupling agents, tacky producers, molecular weight modifiers (for example, mercaptans or polyols) and the like. The composition may further contain a small amount of solvent. These additives are present in an amount of 0.01 to 30 percent by weight, preferably 0.1 to 15 percent by weight based on total amounts of the composition.

The photocurable composition of the present invention is stable at ambient temperature in a dark place. A process for preparing the photocurable composition of the present invention is known. The composition can be obtained by simply mixing whole components.

Light sources for the photocurable composition of the present invention are sunlight, low pressure mercury vapor lamps, high pressure mercury vapor lamps, ultra high pressure mercury vapor lamps, carbon electric arc lamps, metal halide lamps, xenone lamps and the like. The photocurable composition of the present invention can be heated to about 30 to 200 °C before, during or after exposing to light to reduce curing time, if necessary. The exposure to light can be carried out in the air, or under an inert gas such as nitrogen, carbon dioxide or incombustible gas.

The photocurable composition of the present invention is exposed to light to cure. When the composition is cured in a thick film having more than about 10 mm, the cured film has excellent transparency. The composition, therefore, is suitable for a sealing composition or a coating composition for electronic elements, especially light emission elements. As the composition of the present invention is uniformly cured in both surface layer and internal parts, the cured composition has excellent adhesion properties, flexibility, and impact resistance. The composition, therefore, can be usuful for other usages, such as protection, ornament, insulation, coating, inks, sealing, photoresists, solder resists, adhesion and laminate.

The present invention is illustrated by the following examples. In the examples, all parts and percents are by weight unless otherwise specified.

Production exmaple 1

Preparation of Modified epoxy compound 1

Modified epoxy compound 1 was prepared from the following ingredients:

| Ingredients | Part by weight |
|---|---|
| Charge A | |
| Bisphenol A type epoxy compound[1] | 136.2 |
| Dioxane | 120.0 |
| Charge B | |
| Isocyanatoethyl methacrylate | 46.5 |
| Dioxane | 45.0 |
| Charge C | |

Dibutyl tin dilaurate 0.3

Charge D

Hydroquinone 0.2

[1] Epoxy equivalent is 450 to 500, number of hydroxyl group is 2/molecule, and molecular weight is 900 to 1,000.

Charge A was charged in a 500 ml four-neck flask equipped with a stirrer, a thermometer, a decanter, a dropping funnel and an introducing tube for nitrogen gas. It was heated to an inside temperature of 120 °C and refluxed for two hours to remove water. After the mixture was allowed to cool to 80 °C, Charge C and D were added under nitrogen blanket. Charge B was then added dropwise over 30 minutes. After about 30 minute, the termination of the reaction was verified by disappearance of NCO absorption of IR spectrum. The reaction mixture was evaporated using a rotary evaporator to obtain Modified epoxy compound 1 having nonvolatile content of 100 %.

Production exmaple 2

Preparation of Modified epoxy compound 2

Modified epoxy compound 1 was prepared from the following ingredients:

| Ingredients | Part by weight |
|---|---|
| Charge A | |
| Bisphenol A type epoxy compound[1] | 136.2 |
| Dioxane | 120.0 |
| Charge B | |
| Methacryloyl isocyanate | 33.3 |
| Dioxane | 45.0 |

[1] Epoxy equivalent is 450 to 500, number of hydroxyl group is 2/molecule, and molecular weight is 900 to 1,000.

Charge A was treated as generally described in Production example 1. Charge B was then added dropwise over 30 minutes. After about 30 minute, the termination of the reaction was verified by disappearance of NCO absorption of IR spectrum. The reaction mixture was evaporated using a rotary evaporator to obtain Modified epoxy compound 2 having nonvolatile content of 100 %.

Production exmaple 3

Preparation of Modified epoxy compound 3

Modified epoxy compound 1 was prepared from the following ingredients:

| Ingredients | Part by weight |
|---|---|
| Charge A | |
| Bisphenol A type epoxy compound[1] | 136.2 |
| Dioxane | 120.0 |
| Charge B | |
| Vinyl isocyanate | 20.7 |
| Dioxane | 20.0 |
| Charge C | |
| Dibutyl tin dilaurate | 0.3 |
| Charge D | |
| Hydroquinone | 0.2 |

[1] Epoxy equivalent is 450 to 500, number of hydroxyl group is 2/molecule, and molecular weight is 900 to 1,000.

Charge A was treated as described in Production example

1. After the mixture was allowed to cool to 55 °C, Charge C and D were added under nitrogen blanket. Charge B was then added dropwise over 30 minutes. After about 2 hours, the 2 ermination of the reaction was verified by disappearance of NCO absorption of IR spectrum. The reaction mixture was evaporated using a rotary evaporator to obtain Modified epoxy compound 3 having nonvolatile content of 100 %.

Example 1

A photocurable composition was prepared as follow.

Two parts by weight of 50 % aromatic sulfonium salt solution in propylene carbonate and 1 part by weight of 2-hydroxy-2-methyl-1-phenyl-propane-1-on were added to 100 parts of Modified epoxy compound 1 prepared in Production example 1 and mixed at room temperature to form a photocurable composition.

The photocurable composition was poured in a polypropylene cylindrical container having a inside diameter of 10 mm and a height of 10 mm. The composition was exposed with an ultra high-pressure mercury vapor lamp (ORC polymer-printer HMW-40-1) at 400 $mJ/cm^2$ to obtain a cured article. Transparency of the cured article was evaluated. Evaluation was made based on the following criteria.

P; Transparent

L; Translucent

O; Almost opaque

The result was shown in Table 1.

Example 2

A photocurable composition was prepared as follow.

Two parts by weight of 50 % aromatic sulfonium salt solution in propylene carbonate and 1 part by weight of 2-hydroxy-2-methyl-1-phenyl-propane-1-on were added to 100 parts of Modified epoxy compound 2 prepared in Production example 2 and mixed at room temperature to form a photocurable composition.

The same test as Example 1 was carried out and the result was shown in Table 1.

Example 3

A photocurable composition was prepared as follow.

Two parts by weight of 50 % aromatic sulfonium salt solution in propylene carbonate and 1 part by weight of 2-hydroxy-2-methyl-1-phenyl-propane-1-on were added to a mixture of 6.7 parts by weight of Modified epoxy compound 1 prepared in Production example 1 and 33 parts by weight of 3,4-epoxycyclohexylmethyl-3',4'-epoxycyclohexane carboxylate, and mixed at room temperature to form a photocurable composition.

The same test as Example 1 was carried out and the result was shown in Table 1.

Example 4 to 6

The components shown in Table I were employed to prepare photocurable compositions.

The same test as Example 1 was carried out and the result was shown in Table 1.

Exmaple 7

Two parts by weight of 50 % aromatic sulfonium salt solution in propylene carbonate and 1 part by weight of 2-hydroxy-2-methyl-1-phenyl-propane-1-on were added to a mixture of

6.7 parts by weight of Modified epoxy compound 2 prepared in Production example 2 and 33 parts by weight of 3,4-epoxycyclohexylmethyl-3',4'-epoxycyclohexane carboxylate, and mixed at room temperature to form a photocurable composition.

The same test as Example 1 was carried out and the result was shown in Table 1.

Example 8

A photocurable composition was prepared as generally described in Example 7 with the exception that Modified epoxy compound 3 prepared Production example 3 was employed instead of Modified epoxy compound 2.

The same test as Example 1 was carried out and the result was shown in Table 1.

Example 9

A photocurable composition was prepared as generally described in Example 1 with the exception that diphenyl iodonium tetrafluoroborate was employed instead of 50 % aromatic sulfonium salt solution in propylene carbonate.

The same test as Example 1 was carried out and tho result was shown in Table 1.

Comparative example 1

For comparison, a photocurable composition was prepared as follow:

3.6 parts by weight of 50 % aromatic sulfonium salt solution in propylene carbonate and 2.0 parts by weight of benzoin isopropyl ether were added to a solution containing 69 parts of 3,4-epoxycyclohexylmethyl-3',4'-epoxycyclohexane

carboxylate and 31 parts of trimethylolpropane triacrylate, and mixed at room temperature to form a photocurable composition.

The same test as Example 1 was carried out and the result was shown in Table 1.

Comparative example 2 to 4

For comparison, photocurable compositions were prepared employing the components shown in Table 1.

The same test as Example 1 was carried out and the result was shown in Table 1.

Table 1

| Component (parts) | Example 1 | 2 | 3 | 4 | 5 | 6 | 7 | 8 | 9 | Comparative Example 1 | 2 | 3 | 4 |
|---|---|---|---|---|---|---|---|---|---|---|---|---|---|
| Production example 1 | 100 | – | 67 | 10 | 90 | 31 | – | – | 100 | – | – | – | – |
| Epoxy compound 1) | – | – | 33 | 90 | 10 | 69 | 67 | 67 | – | 69 | – | – | – |
| Production example 2 | – | 100 | – | – | – | – | 33 | – | – | – | – | – | – |
| Production example 3 | – | – | – | – | – | – | – | 33 | – | – | – | – | – |
| Epoxy compound 2) | – | – | – | – | – | – | – | – | – | – | 25 | – | – |
| " 3) | – | – | – | – | – | – | – | – | – | – | – | 35 | – |
| " 4) | – | – | – | – | – | – | – | – | – | – | – | – | 25 |
| " 5) | – | – | – | – | – | – | – | – | – | 31 | – | – | – |
| " 6) | – | – | – | – | – | – | – | – | – | – | 75 | 65 | 75 |
| Onium compound 7) | 2 | 2 | 2 | 2 | 3 | 3.6 | 2 | 2 | – | 3.6 | 0.8 | 1 | 0.8 |
| " 8) | – | – | – | – | – | – | – | – | 2 | – | – | – | – |
| Sensitizer 9) | 1 | 1 | 1 | 1 | 1 | – | 1 | 1 | 1 | – | – | – | – |
| " 10) | – | – | – | – | – | 2.0 | – | – | – | 2.0 | 0.4 | 2.0 | 3.7 |
| Transparency | P | P | P | P | P | P | P | P | P | L | L | O | O |

Epoxy compound 1) : 3,4-Epoxycyclohexylmethyl-3',4'-epoxycyclohexane carboxylate
" 2) : Propylene glycol diglycidyl ether
" 3) : 1,6-Hexanediol diglycidyl ether
" 4) : Polypropylene glycol diglycidyl ether
" 5) : Trimethylolpropane triacrylate
" 6) : A diacrylate of bisphenol A ethyleneoxide adduct
Onium Compound 7) : 50% aromatic sulfonium salt in propylene carbonate
" 8) : Diphenyl iodonium tetrafluoroborate
Sensitizer 9) : 2-Hydroxy-2-methyl-1-phenyl-propane-1-on
" 10) : Benzoin isopropyl ether

CLAIMS

1. A photocurable composition comprising;
(a) an epoxy compound modified with a compound having a polymerizable ethylenically unsaturated group,
(b) a polymerization initiator capable of initiating cationic polymerization by exposing to light, and
(c) a sensitizer.

2. The photocurable composition of Claim 1 wherein the modification is carried out by reacting the unmodified epoxy compound having a hydroxyl group with an isocyanate compound containing a polymerizable ethylenically unsaturated group.

3. The photocurable composition of Claim 2 wherein the unmodified epoxy compound is bisphenol A type epoxy compound.

4. The photocurable composition of Claim 2 wherein the isocyanate compound is vinyl isocyanate, an isocyanatoalkyl (meth)acrylate, (meth)acryloyl isocyanate.

5. The photocurable composition of Claim 1 wherein the modified epoxy compound is prepared by reacting the epoxy compound having hydroxyl groups with a product of reacting a hydroxyalkyl (meth)acrylate with diisocyanate.

6. The photocurable composition of Claim 1 wherein the modified epoxy compound is prepared by reacting the epoxy compound having hydroxyl groups with a (meth)acryl halide in the presence of a tertiary amine.

7. The photocurable composition of Claim 1 wherein the

modified epoxy compound is combined with an unmodified epoxy compound.

8. The photocurable composition of Claim 1 wherein the polymerization initiator is an onium compound which release a material having a capability of initiating cationic polymerization.

9. The photocurable composition of Claim 8 wherein the onium compound is represented by following formula:

$$[(R)_a(R^1)_b(R^2)_cX]_d^+[MQ_e]^{-(e-f)}$$

Wherein R represents a monovalent aromatic organic group, $R^1$ represents a monovalent aliphatic organic group selected from an alkyl group, a cycloalkyl group or a substituted alkyl group, $R^2$ represents a polyvalent organic group having heterocyclic or combined cyclic structure, selected from an aliphatic group or an aromatic group, X represents an element selected from diazonium, halogen, nitrogen, arsenic , antimony, bismuth, sulfur, selen or tellurium, a is an integer of 0 to 3, b is an integer of 0 to 2, c is 0 or 1 and a + b + c is 3 which is equal to a valency of X, d is equal to (e-f), f is an integer of 2 to 7 equal to a valency of M, Q represents a halogen atom and e is an integer up to 5 and more than f.

10. The photocurable composition of Claim 1 wherein the polymerization initiator is present in the composition of the present invention in an amount of 0.01 to 10 parts by weight based on 100 parts by weight of the modified epoxy compound.

11. The photocurable composition of Claim 1 wherein the sensitizer is present in the composition of the present

invention in an amount of 0.01 to 10 parts by weight based on 100 parts by weight of the modified epoxy compound.

12. A cured article prepared by curing the photocurable composition of Claim 1.